(19) Europäisches Patentamt
European Patent Office
Office européen des brevets

(11) Veröffentlichungsnummer: **0 157 384 B1**

(12) # EUROPÄISCHE PATENTSCHRIFT

(45) Veröffentlichungstag der Patentschrift: **18.01.95**

(21) Anmeldenummer: **85103807.5**

(22) Anmeldetag: **29.03.85**

Die Akte enthält technische Angaben, die nach dem Eingang der Anmeldung eingereicht wurden und die nicht in dieser Patentschrift enthalten sind.

(51) Int. Cl.6: **C07H 17/02**, C07H 17/075, C07H 17/00, C07H 17/04, C07D 311/82, C07F 9/09, C07K 5/08, C07C 317/00, C12Q 1/44

(54) **Substrate für Hydrolasen, Verfahren zu ihrer Herstellung und ihre Verwendung.**

(30) Priorität: **06.04.84 DE 3412939**

(43) Veröffentlichungstag der Anmeldung:
**09.10.85 Patentblatt 85/41**

(45) Bekanntmachung des Hinweises auf die Patenterteilung:
**18.01.95 Patentblatt 95/03**

(84) Benannte Vertragsstaaten:
**AT BE CH DE FR GB IT LI LU NL SE**

(56) Entgegenhaltungen:
**EP-A- 0 031 941**
**EP-A- 0 074 761**
**EP-A- 0 156 347**
**US-A- 3 378 463**
**US-A- 3 950 322**

(73) Patentinhaber: **Biomed Labordiagnostik GmbH**
**Bruckmannring 28**
**D-85764 Oberschleissheim (DE)**

(72) Erfinder: **Wallenfels, Kurt, Prof. Dr.**
**Vordere Steige 7**
**W-7800 Freiburg (DE)**
Erfinder: **Fathy, Ahmed Morhaf, Dr.**
**Ritterstrasse 3**
**W-7600 Offenburg (DE)**

(74) Vertreter: **Schwabe - Sandmair - Marx**
**Stuntzstrasse 16**
**D-81677 München (DE)**

**EP 0 157 384 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BIOCHIMICA ET BIOPHYSICA ACTA, Band 526, 1978, Seiten 489-506 Elsevier/North-Holland Biomedical Press, NL; A.B. ROY:"The sulphatase of ox liver, XXI: kinetic studies of the substrate-induced inactivation of sulphatase A"

AUSTRALIAN JOURNAL OF CHEMISTRY; Band 23, Nr. 4, April 1970 Seiten 847-852 A. JERFY et al.: "Sulphated derivatives of some hydroxy-pyridines"

CHEMICAL ABSTRACTS, Band 78, Nr. 13, 2 April 1973, Seite 191, Zusammenfassung 81447m, Columbus, Ohio, US; Y. MURAKAMI et al.: "Enzymic and nonenzymic hydrolyses of heteroaromatic phosphates",

ANALYTICA CHIMICA ACTA, vol. 163, 1984, pages 167-72 Elsevier Science Publishers B.V. Amsterdam, NL; J. Hofman et al.: "Immobilized enzyme kinetics analyzed by flow-through microfluorimetry resorfin-beta-D-galactopyranoside as a new fluoroenic substrate for beta-galactosidase"

## Beschreibung

Die Erfindung betrifft Substrate zur Bestimmung von Hydrolasen, ein Verfahren zu ihrer Herstellung, ihre Verwendung zur Bestimmung von Hydrolasen und dazu geeignete Mittel.

Der Nachweis oder die Bestimmung von Hydrolasen sind von Interesse für die Erkennung und Verlaufskontrolle verschiedener Krankheiten. Beispiele sind die Bestimmung der Amylase für die Pankreas-Diagnostik und der sauren Phosphatase für die Erkennung des Prostata-Karzinoms (Übersicht in R.J. Haschen, Enzymdiagnostik, Gustav Fischer Verlag, Stuttgart, 1970), von Esterasen als Indikatoren für Leukozyten (EP-A-12957) oder von Phosphatase oder Galactosidase im Enzymimmunoassay als Indikatoren für immunologische Bestimmungsverfahren.

Zur Zeit werden Bestimmungen von Hydrolasen, die ein pH-Optimum unter 7 haben, im allgemeinen photometrisch mit einer Zweipunktmessung durchgeführt.

Zweipunktbestimmungen benötigen allgemein eine längere Zeit als kinetisch durchführbare Bestimmungen und sind darüberhinaus auf den weit verbreiteten Analysenautomaten nicht anwendbar, so daß nur die aufwendige manuelle Bestimmung möglich ist. Außerdem weisen sie eine geringere Präzision auf als kinetische Verfahren und sind schlechter kontrollierbar, was zu falschen Ergebnissen führen kann.

Die wenigen, kinetisch durchführbaren Bestimmungen von Hydrolasen mit saurem pH-Optimum benötigen mehrstufige Reaktionen, wie z. B. für die saure Phosphatase in der deutschen Patentschrift 21 15 748 beschrieben, oder verwenden fluorogene Substrate, wie z. B. Derivate des 4-Methylumbelliferon. Mehrstufige Reaktionen führen jedoch zu Störungen der Bestimmung durch Verunreinigungen in den Reagenzien, vor allem in den Hilfsenzymen, und zu Konkurrenzreaktionen mit Bestandteilen biologischer Flüssigkeiten. Außerdem verteuern sie das Verfahren. Fluoreszierende Stoffe können nur mit speziellen Geräten bestimmt werden, sind schlecht standardisierbar und störanfällig, was zu schlechten Präzisionen und falschen Resultaten führt. Außerdem können fluorogene Substrate nicht in der üblicherweise erforderlichen Sättigungskonzentration für die zu bestimmende Enzymaktivität eingesetzt werden, da unter diesen Bedingungen die Fluoreszenz des Reaktionsproduktes durch die Absorption des Substrates zum größten Teil wieder gelöscht wird. Auch dies führt zu falschen Ergebnissen bzw. aufwendigen Standardisierungen.

Es sind auch bereits Verfahren zur Bestimmung der $\alpha$-Amylase-Aktivität mit p-Nitrophenyl-$\alpha$-maltodextrinen bekannt, zum Beispiel Testomar®-Amylase, Behringwerke AG, Bundesrepublik Deutschland, worin die Bestimmung kinetisch bei pH 7 durchgeführt wird, was den Nachteil der starken Abhängigkeit des Extinktionskoeffizienten des freigesetzten Nitrophenols vom pH-Wert hat und den Test störanfällig macht. Außerdem wird bei der Nitrophenylgruppe als Chromogen etwa 50% der Empfindlichkeit eingebüßt, da der molare Extinktionskoeffizient des Nitrophenol bei pH 7 nur halb so groß ist wie beispielsweise bei pH 9. Bei pH 9 ist jedoch eine Aktivitätsbestimmung der Amylase nicht möglich, da diese unter diesen Bedingungen ihre Aktivität verliert.

Außerdem sind bisher alle kinetisch durchführbaren Amylase-Bestimmungen (DOS 27 52 01, DOS 27 31 372, DOS 28 22 364, DOS 30 00 292, JP 113138) mehrstufige Reaktionen, die ein oder mehrere Hilfsenzyme benötigen. Dies ist mit den oben bereits beschriebenen Nachteilen verbunden.

Bei Bestimmungen von Spurenkomponenten biologischer Flüssigkeiten spielt die Empfindlichkeit des Nachweissystems eine entscheidende Rolle. So wurden in jüngster Zeit neben dem Radioimmunoassay Enzymimmunoassays entwickelt, deren zum Beispiel mit Galactosidase gekoppelte Indikator-Reaktion den Nachweis von Spurenproteinen im menschlichen Serum erlaubt. Die bisherigen Methoden haben jedoch alle den Nachteil einer Inkubationszeit von einer bis mehreren Stunden. Durch eine größere Empfindlichkeit der Indikatorreaktion könnte entweder die Inkubationszeit reduziert oder bei gleicher Inkubationszeit die Empfindlichkeit entsprechend gesteigert werden.

Esterasen sind als Indikatoren für Leukozyten diagnostisch interessant. Neben der Spezifität ist heutzutage auch ein großes Maß an Sensitivität erforderlich, um das Ergebnis bereits nach wenigen Minuten Reaktionszeit ablesen zu können. Bei visuell auswertbaren Testsystemen wie zum Beispiel Teststreifen werden Farbstoffe benötigt, die für das menschliche Auge optimale Farbanzeigen (rot, grün, blau) liefern. Die gelbe Farbe des Nitrophenols oder Nitranilins ist hier sehr ungünstig. Die erreichbare Sensitivität hängt somit entscheidend von dem chromogenen Molekülteil des Substrates ab.

Die chromogenen Substrate des Standes der Technik sind entweder wegen der entstehenden Farbe (gelb) oder wegen zu geringer Reaktivität zur visuellen Auswertung nicht geeignet. So werden z. B. mit den chromogenen Aminosäure-Estern aus den Patentschriften EP 0007 407 (Sulfophthaleine), 0008 438 (Azofarbstoffe), 0012 957 (Indoxyl) oder DE 30 05 845 (Indoaniline), die alle sehr gut visuell erkennbare Farben geben, in einer für Schnelltests annehmbaren Entwicklungszeit von etwa 2 min Anzeigeempfindlichkeiten bis herab zu 2 bis 5 Leukozyten/µl erhalten. Diagnostisch ist hingegen ein sehr viel niedrigerer Grenzwert anzustreben. Eine Erhöhung der Sensitivität wird bei diesen Substraten durch einen zusätzlichen Einsatz

von Acceleratoren erreicht.

Im US-Patent 3378463 wird die Fluoreszenz von Esterase-Substraten mit Chromogenen von Typ Indoxyl und Resorufin beschrieben; die jedoch nur bei Auswertung mittels entsprechender Geräte (Fluorometer) brauchbar sind. Von Interesse ist die hier in Spalte 5; Zeilen 61 bis 65; getroffene Feststellung; daß entsprechende Indoxyl-Derivate viel schneller gespalten werden als die Resorufin-Derivate. In Verbindung mit dem zuvor Gesagten war demnach anzunehmen; daß die Resorufin-Ester zum Beispiel beim Nachweis von Esterasen aus Leukozyten eine noch geringere Sensitivität aufweisen würden.

Ein weiterer Nachteil der in jener Patentschrift beschriebenen Substrate liegt in ihrer Unspezifität. Mit Resorufin-Acetat und Butyrat reagieren sowohl Esterasen wie Cholinesterase, Acylase, Lipase oder saure Phosphatase, als auch Proteasen, wie Chymotrypsin (Tabelle III der US-Patentschrift).

Weiterhin beschreibt die US-A-3,950,322 fluorhaltige Substrate, die ein Ketosid enthalten, das zwischen einer N-Acylneuraminsäure und einem stark fluoreszierenden Material enthaltend eine phenolische Gruppe gebildet wird.

Auch aus der EP-A-74 761 sind chromogene oder fluorgene Substrate vom Peptid-Typ bekannt, die für die Bestimmung von Endotoxin geeignet sind.

Es bestand demnach die Aufgabe, empfindliche Indikatoren für Enzym-Reaktionen zur Verfügung zu stellen, wobei der durch die Hydrolyse des zu bestimmenden Enzymes freigesetzte Farbstoff (als Farbstoffe werden von uns Verbindungen mit einer Absorption zwischen 300 und 760 $\mu$m verstanden) einen möglichst niedrigen $pK_a$-Wert und einen möglichst hohen molaren Extinktionskoeffizienten des Anions hat, um dadurch eine kinetische, mittels Analysenautomaten durchführbare Bestimmung von Hydrolasen zu ermöglichen oder eine empfindlichere Indikatorreaktion für Enzymimmunoassays aufbauen zu können. Es bestand weiterhin die Aufgabe, Enzym-Substrate zur Verfügung zu stellen, die spezifisch mit nur einem Enzym reagieren und somit in einem Enzymgemisch, wie es in biologischen Flüssigkeiten vorliegt, eine spezifische Bestimmung eines Enzyms oder einer Gruppe von Enzymen erlauben.

Es wurde nun überraschend gefunden, daß Verbindungen der allgemeinen Formel A-O-B, worin A den Rest des Ions $A\text{-}O^-$ einer heterocyclischen Verbindung A-OH mit saurem $pK_a$-Wert darstellt, dessen Extinktion nach Hydrolyse von A-O-B photometrisch gemessen wird, während B der Rest einer Verbindung B-OH ist, der die Verbindung für die Reaktion mit einem bestimmten Enzym spezifisch macht, und wobei A-OH

a. eine Verbindung der allgemeinen Formel I

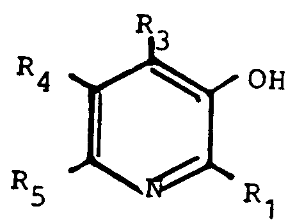

I

worin $R_1$ OH, $NO_2$ oder Halogen und
$R_3$, $R_4$ und $R_5$ H oder $NO_2$ sein können, wobei höchstens zwei $NO_2$ vorhanden sind,
b. eine Verbindung der allgemeinen Formel IV

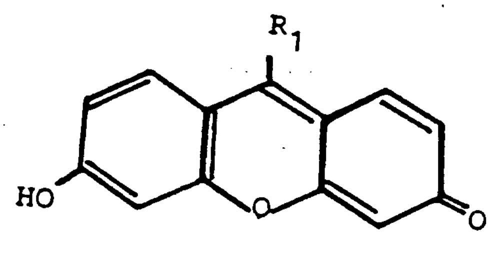

IV

worin $R_1$ Aryl, vorzugsweise Phenyl, CN oder Alkyl mit 1-12, vorzugsweise 1-3, Kohlenstoffatomen sein können, und wobei B-OH ein Zucker oder Zuckerderivat, insbesondere Glucose, ein Maltodextrin, Galactose, Glucuronsäure, N-Acetylglucosamin oder Fucose, eine Aminosäure oder ein Oligopeptid, die eine Schutzgruppe, besonders die Toluolsulfonyl- oder Carbobenzyloxy(CBZ)-Gruppe tragen können,

insbesondere N-Tosylalanin oder CBZ-Glycin, oder eine anorganische Säure, insbesondere Phosphorsäure, wobei Schwefelsäure ausgenommen ist, ist, Substrate mit hoher Nachweisempfindlichkeit für die Bestimmung von Hydrolasen sind.

Gegenstand der Erfindung ist also eine Verbindung der Formel A-O-B wie vorstehend definiert, ein Verfahren zu ihrer Herstellung, sowie ihre Verwendung zur Bestimmung einer Hydrolase.

Für die kinetische Bestimmung von Hydrolasen mit einem pH-Optimum unter 7 sind besonders solche Substrate geeignet, bei deren Hydrolyse ein Farbstoff mit einem $pK_a$-Wert unter 7 freigesetzt wird. Die verwendeten Farbstoffe zeigen bereits bei einem pH-Wert unter 7 eine intensive Farbe und können dementsprechend photometrisch oder mit dem Auge empfindlich nachgewiesen werden.

Es bestand ein Vorurteil gegen die Möglichkeit einer Synthese von Glykosiden von Verbindungen mit niedrigem $pK_a$-Wert, das heißt von vergleichsweise stark sauren Verbindungen, da für solche eine geringe thermodynamische Stabilität zu erwarten gewesen war.

Solche Verbindungen haben eher den Charakter von Anhydriden, die bekanntermaßen thermodynamisch wesentlich instabiler sind als normale Glykoside und Ester.

Zur Überraschung wurde jedoch gefunden, daß Derivate der oben aufgeführten heterocyclischen Stoffe, die niedrige $pK_a$-Werte haben - verglichen mit bisher zur Synthese von Glykosiden und Estern verwendeten isocyclischen Verbindungen wie zum Beispiel 2,4-Dinitrophenylgalactosid -, eine ausreichende kinetische Stabilität haben, um mit zufriedenstellenden Ausbeuten gebildet zu werden.

Eine weitere überraschende Eigenschaft dieser Verbindungen war, daß sie schärfere, teilweise sehr hohe und langwellige Absorptionsbanden aufweisen, die sie als Indikatoren für die weit verbreitete absorptionsphotometrische Messung besonders geeignet machen.

Für die beschriebenen Verbindungen gilt zudem, daß sie von den Hydrolasen, als deren Substrate sie verwendet werden sollen, besonders schnell gespalten werden, obwohl die Farbstoff-Komponenten keine Ähnlichkeit mit den natürlichen analogen Substratanteilen (aglykonischer Teil in Disacchariden oder Stärke, alkoholische Komponente von Estern) aufweisen. Dies war nicht zu erwarten.

Die Substrate mit der Allgemeinformel A-O-B wurden in drei Stufen hergestellt:

1. Herstellung des Teiles AOH, nämlich des Farbstoffs,

2. Herstellung des Teiles BOH, nämlich eines Kohlenhydrats oder Kohlenhydratderivates oder einer Säure und

3. Herstellung des Substrates A-O-B aus AOH und BOH durch eine Methode, die insbesondere bei den Kohlenhydraten und Kohlenhydratderivaten, ein Substrat mit der gewünschten Stereoisomerie liefert.

Die bekannten Farbstoffe können nach folgenden Verfahren hergestellt werden:

Nitro-pyridin-und -cumarinderivate allgemein durch Nitrierung der entsprechenden Ausgangsverbindungen in Schwefelsäurelösung bei einer Temperatur um 0 °C durch Eintropfen einer $HNO_3/H_2SO_4$-Mischung (Shah und Mehta, J. Indian Chem. Soc. (1954), 784; Pechman und Obermüller, Ber. 34 (1901), 660; Chakrawarti, J. Indian Chem. Soc. Ber. 34 (1901), 660; Chakrawarti, J. Indian Chem. Soc. 12 (1935), 791-795; Burton et al., J. Chem. Soc. Perin II (1972), 1953-1958; Brignell et al., J. Chem. Soc. (B) (1968), 1477; de Selems, J. Org. Chem. 33 (1968), 478; Talik und Talik, Rocziniki Chemii, Ann. Soc. Chem. Polonorum 40 (1966), 1187); Resazurin (10-Oxid des 3H,7-Hydroxyphenoxazin-3-on nach Nietzki et al., Ber. der Deut. Chem. Ges. 22 (1889), 3020; Resorufin (3H,7-Hydroxyphenoxazin-3-on) durch Reduktion von Resazurin mit Natrium-Borhydrid; 6-Hydroxy-9-phenyl-3H-xanthen-3-on nach Nathsen, J. Amer. Chem. Soc. 47 (1925), 1079; andere Phenoxazine und Xanthenone nach Stuzka et al., Collection Czechoslov. Chem. Commun. Vol. 34 (1969), 221 oder Kehrman und de Gottrau, Ber. 38 (1905), 2575.

3-H,6-Hydroxy-9-cyanxanthen-3-on, das von uns nachfolgend als Cyanorufin bezeichnet wird, ist neu. Es wurde nach dem in den Beispielen beschriebenen Verfahren hergestellt.

Ebenfalls neu ist 7-Hydroxy-3-nitrocumarin, das durch Ringschluß eines Nitrostyrolderivates hergestellt wurde. Es hat einen Schmelzpunkt von 192-198 °C und bei pH > 8 ein Absorptionsmaximum bei 450 nm, bei pH < 6 liegt das Absorptionsmaximum bei 390 nm.

Die zur Herstellung der Verbindungen der Formel A-O-B durch Reaktion mit A-OH geeigneten, gegebenenfalls Schutzgruppen tragenden, bekannten Derivaten der Kohlenhydrate B-OH wurden nach Methods in Carbohydrate Chemistry, Vol. II, Academic Press 1963, hergestellt. Die mit Schutzgruppen versehenen Aminosäuren oder Oligopeptide wurden gekauft oder nach dem Beispiel 16 (J. Amer. Chem. (1937) 59, 1116-1118) hergestellt.

Die neuen Verbindungen der Formel A-O-B wurden nach an sich bekannten Verfahren aus einer Verbindung der Formel A-OH und einem reaktionsfähigen Derivat einer Verbindung der Formel B-OH hergestellt.

Die Verbindungen der Formel A-O-B können als Substrate zum Nachweis und zur Bestimmung von Hydrolasen verwendet werden. Die Farbstoffe A-OH, die bei der Spaltung freigesetzt werden, haben

wesentliche Vorteile gegenüber denen des Standes der Technik. Während der $pK_a$-Wert des vielfältig verwendeten Farbstoffs p-Nitrophenol, das heißt der pH-Wert, bei dem die Hälfte des p-Nitrophenols dissoziiert als Phenolat-Anion vorliegt, bei 7 liegt, ist der $pK_a$-Wert der von uns verwendeten Farbstoffe A-OH kleiner als 6 (Tabelle 1). Dies bedeutet, daß bereits im pH-Bereich unter 7 eine gegenüber p-Nitrophenol wesentlich höhere Extinktion bei gleicher Molarität vorliegt, so daµ die aus diesen Farbstoffen neu hergestellten Substrate somit wesentlich empfindlicher gemessen werden können.

Im folgenden werden die Vorteile einer Verwendung der neuen Substrate gegenüber denen des Standes der Technik beispielhaft beschrieben.

Die Aktivität der α-Glucosidase in Human-Harn kann mit dem neuen Substrat 2-H,7-O-(α-D-Glucopyranosyl)-4-methyl-8-nitro-benzpyran-2-on (Verbindung S1, siehe Beispiel 1) sehr empfindlich im kinetischen Test bestimmt werden. Da das pH-Optimum der α-Glucosidase bei 4 bis 6 liegt, kann mit dem bisher üblichen Substrat p-Nitrophenyl-α-glucopyranosid die Aktivität nicht kinetisch gemessen werden, weil das freigesetzte Nitrophenol bei 405 nm nur eine sehr geringe Extinktion aufweist (Tabelle I).

## Tabelle 1

Extinktionskoeffizienten von beispielhaften Farbstoffen bei unterschiedlichen pH-Werten

| Farbstoff | $pK_a$ | Wellen-länge (nm) | Extinktionskoeffizient ($l \cdot mol^{-1} \cdot cm^{-1} \cdot 10^3$) pH 4,0 | 5,0 | 6,0 | 7,1 | 10 |
|---|---|---|---|---|---|---|---|
| 4-Methyl-8-nitro-benz-pyran-2-on | 4,45 | b) 350 | 8,50 | 15,0 | 18,80 | 19,4 | 19,4 |
| | | c) 365 | 5,80 | 11,3 | 16,10 | 16,25 | 16,30 |
| 3-Hydroxy-2-nitropyri-din | 5,1 | 405 | 0,50 | 2,32 | 5,09 | 5,80 | 5,90 |
| Resorufin | 5,95 | b) 578 | 0,75 | 5,7 | 22,80 | 39,6 | 41,000 |
| | | c) 574 | a) 1,00 | a) 7,4 | a) 29,64 | 49,87 | 55,42 |
| 3-Hydroxy-4-nitro-2-chlor-pyridin | 3,1 | 405 | 4,93 | 5,34 | 5,40 | 5,48 | 5,4 |
| 3-Hydroxy-2-nitro-6-chlor-pyridin | 3,9 | 365 | 7,84 | 11,87 | 12,9 | 13,2 | 13,2 |
| p-Nitro-phenol | 7,0 | 405 | 0,20 | 0,40 | 1,50 | 9,7 | 18,30 |
| o-Nitro-phenol | 7,08 | 405 | 0,47 | 0,48 | 0,75 | 2,34 | 4,01 |

a) berechnet b) Monochromator c) Spektrallinienphotometer mit Quecksilberlampe

Ein Testansatz mit diesem Substrat muß also nach einer bestimmten Reaktionszeit durch Zusätze wie Bicarbonat alkalisiert werden, um eine meßbare Extinktion zu erreichen.

Demgegenüber konnte mit der Verbindung S1 als Substrat in normalem Humanharn eine Extinktionsdifferenz pro Minute von 40 mE im kinetischen Test gemessen werden, womit die Automatisierung dieser Bestimmung möglich ist.

Es ist bekannt, daß im Harn Hemmstoffe auftreten können, die die Aktivität der Glucosidase beeinflussen. In diesem Falle muß durch geeignete Maßnahmen, wie zum Beispiel Dialyse oder Gelfiltration der Hemmstoff entfernt werden. Ein Einsatz von gereinigter β-Glucosidase zeigte, daß das Substrat S1 ein spezifisches Substrat für die α-Glucosidase ist, da es von der β-Glucosidase nicht gespalten wird.
Eine weitere Möglichkeit der kinetischen Bestimmung der α-Glucosidase-Aktivität besteht in der Verwendung von 3-H,7-O-(α-Glucopyranosyl)-phenoxazin-3-on (Resorufin-α-D-Glucopyranosid). Der $pK_a$-Wert des Resorufins liegt zwar bei 5,9, durch den sehr hohen molaren Extinktionskoeffizienten bei 578 nm kann jedoch beispielsweise bei pH 6 die Harnglucosidase mit ausreichender Empfindlichkeit bestimmt werden. Für die Durchführung bei 37 °C wurden bei Verwendung von 400 µl Reagenz und 200 µl Harn eine Extinktionsdifferenz pro Minute von 136 mE gefunden. Die Messung bei 578 nm hat den Vorteil, daß die gefärbten Inhaltsstoffe des Harns nicht stören. Auch hier zeigte der Einsatz von β-Glucosidase, daß Resorufin-α-Glucopyranosid ein spezifisches Substrat für die α-Glucosidase ist.

Die Bestimmung der β-Glucosidase-Aktivität im menschlichen Harn ist mit herkömmlichen Testen aufgrund von deren geringen Empfindlichkeit bisher nicht möglich gewesen. Mit 3-O-(β-D-Glucopyranosyl-)3-hydroxy-2-nitropyridin (Verbindung S5) und p-Nitrophenyl-β-D-glucopyranosid wurde die β-Glucosidase-Aktivität vergleichend gemessen. Die Ergebnisse sind in Tabelle 2 gegenübergestellt und belegen, daß mit dem neuen Substrat S5 eine etwa 7-fach größere Extinktionsänderung pro Minute gefunden wurde.

Tabelle 2

| Bestimmung der β-Glucosidase-Aktivität in einer Harnprobe | |
|---|---|
| Substrat | T = 25 °C delta mE/min |
| p-Nitrophenylderivat | 47,6 |
| S5 | 332 |

Für die α-Amylase-Bestimmung wurden die Extinktionsänderungen mit folgenden neuen Substraten vergleichend gemessen: 2H,7-O(α-D-Maltotriosyl)-4-methyl-8-nitrobenzpyran-2-on (Verbindung S10), der entsprechenden Maltotetraosylverbindung (Verbindung S11) sowie o-Nitrophenyl-α-maltotriosid.

Mit dem Substrat S 11 wurde eine etwa 14 mal und mit dem Substrat S 10 sogar eine 32 mal größere Extinktionsänderung als mit dem Nitrophenylderivat gemessen (Tabelle3).

Tabelle 3

| Bestimmung der α-Amylase-Aktivität in einer Harnprobe ohne Hilfsenzyma α-Glucosidase | |
|---|---|
| Substrat | delta mE/min |
| Nitrophenylderivat | 20 |
| S11 | 134 |
| S10 | 635 |

Diese hohen Extinktionsänderungen mit den neuen Substraten werden ohne Beteiligung der α-Glucosidase als Hilfsenzym erreicht, das in den bisherigen Testsystemen für die Freisetzung des Farbstoffs erforderlich ist. Damit entfallen Reagenzkosten und weitere Schwierigkeiten, die alle bisherigen kinetischen Teste für die Amylase-Bestimmung aufweisen, nämlich die nichtlineare Anfangsphase der Kinetik, die sich im allgemeinen über mehrere Minuten erstreckt. Bei Verwendung der neuen Substrate verläuft dagegen die Kinetik von Anfang an linear. Damit wird eine wesentlich schnellere Messung manuell und vor allem an Analysenautomaten möglich.

Mit 3-O-(α-D-Maltotriosyl)-3-hydroxy-2-nitropyridin (Verbindung S12) konnten ebenfalls ausreichend hohe Extinktionsänderungen im Vergleich zum Stand der Technik ohne Beteiligung des Hilfsenzyms α-Glucosidase und bei sofortiger linearer Kinetik erreicht werden.

Unter Verwendung von 3-O-(α-D-Galactopyranosyl)-3-hydroxy-2-nitropyridin konnte die Aktivität der α-Galactosidase im menschlichen Harn kinetisch bestimmt werden. Dazu wurden zu 1 ml gepufferter Substrat-Lösung 0,2 ml zehnfachkonzentrierter normaler Harn gegeben und die Extinktionsänderung bei 405 nm und 22 °C verfolgt. Unter diesen Bedingungen wurde eine Extinktionsdifferenz von 1,2 mE/min gemessen. Dieses Substrat wird durch β-Galactosidase nicht gespalten.

Die β-Galactosidase-Aktivität konnte im menschlichen Harn unter Verwendung von 2H,7-O-(β-D-Galactopyranosyl)-4-methyl-8-nitro-benzpyran-2-on (Verbindung S 15) kinetisch gemessen werden. Eine Extinktionsänderung von 14 mE/min wurde bei Verwendung von 200 µl gepufferter Substrat-Lösung und 200 µl Harn normaler Konzentration bei 23 °C und 366 nm registriert. Das Substrat wird von α-Galactosidase nicht gespalten. Auch bei Verwendung von Resorufin-β-D-galactopyranosid (Verbindung S6) konnte die β-Galactosidase im menschlichen Harn kinetisch bestimmt werden. Es wurde hierbei eine Extinktionsänderung von 3,8 mE/min bei 37 °C, 578 nm, erreicht.

Die Aktivität der β-Glucuronidase im menschlichen Harn konnte mit 2H-7-O-(β-D-Glucoronopyranosyl)-4-methyl-8-nitrobenzpyran-2-on (Verbindung S9) kinetisch bestimmt werden. Hierbei wurden zu 0,6 ml gepufferter Substrat-Lösung 0,1 ml Harn-Konzentrat gegeben und die Aktivität bei pH 5; 30 °C und 366 nm verfolgt. Es wurde unter diesen Bedingungen eine Extinktionsdifferenz von 14 mE/min gemessen.

Die Bestimmung ist somit um ein Vielfaches empfindlicher als mit dem herkömmlichen p-Nitrophenyl-Substrat (Tabelle 4).

Tabelle 4

| Bestimmung von β-Glucuronidase in Human-Harn bei pH 5 | |
|---|---|
| Substrat | delta mE/min |
| Verbindung S9 | 14 |
| p-Nitrophenyl-Substrat | 0,5 |

Mit N-Acetyl-β-Glucosaminidase aus Rinderniere wurden die Substrate p-Nitrophenyl-N-acetylamino-glucosid und 2H,7-O-(2-Acetamido-2-deoxy-β-D-glucopyranosyl)-4-methyl-8-nitrobenzpyran-2-on (Verbindung S8) in ihrer Empfindlichkeit miteinander verglichen. Zu 0,5 ml Substrat-Lösung wurden 0,1 ml einer 1:101 verdünnten Enzym-Lösung (Fa. Sigma) gegeben und die Extinktionsänderung bei 22 °C und 405 oder 365 nm bestimmt. Die Ergebnisse sind in Tabelle 5 zusammengestellt.

Die Extinktionsänderung ist bei dem eingestellten pH von 4,5 für das S8 etwa 36fach größer als mit p-Nitrophenylacetylamino-glucosid.

Mit S8 kann auch die Aktivität in Humanharn (10fach konzentriert) kinetisch bestimmt werden. Die Bestimmung mit 0,5 gepufferter Substrat-Lösung und 0,1 ml zehnfach konzentriertem normalen Harn brachte bei 37 °C und 365 nm eine Extinktionsänderung von 23 mE/min bei pH 5,5 und 14 mE/min bei pH4,5. Diese Extinktionsänderungen sind so groß, daß die Aktivität auch in dem gleichen Probevolumen eines unverdünnten Harns noch mit ausreichender Empfindlichkeit kinetisch gemessen werden konnte.

Tabelle 5

| Bestimmung der N-Acetylglucosaminidase | |
|---|---|
| Substrat | delta mE/min |
| p-Nitrophenyl-Substrat | 7,6 |
| Verbindung S8 | 266 |

Mit 2H,7-O-4-Methyl-8-nitro-benzpyran-2-on-phosphat Verbindung S13) konnte die Aktivität der sauren Phosphatase wesentlich empfindlicher kinetisch bestimmt werden als mit Nitrophenylphosphat. Die Bestimmung wurde in Seminal-Plasma, 1:101 verdünnt, mit beiden Substraten durchgeführt. Zu 1 ml einer gepufferten Substrat-Lösung, pH 4,8, wurden 0;01 ml verdünnter Probe gegeben und die Extinktionsänderung bei 405 oder 365 nm kinetisch verfolgt.

Wie Tabelle 6 zeigt, ist die Extinktionsänderung mit S13 etwa um das 13fache größer als für Nitrophenylphosphat.

Tab. 6

| Substrat | delta mE/min | Verhältnis der Extinktionsänderungen |
|---|---|---|
| Verbindung S 13<br>p-Nitrophenylphosphat | 97,5<br>7,5 | 13<br>1 |

Gegenstand der Erfindung ist auch ein diagnostisches Mittel zum Nachweis esterolytischer Enzyme, insbesondere zum Nachweis der in Leukozyten vorhandenen Esterasen bestehend aus einem saugfähigen Träger, einer Filmschicht, einer losen oder verpreßten Pulvermischung, einem Lyophilisat oder einer Lösung, enthaltend eine oder mehrere Verbindungen der Formel A-O-B wie vorstehend definiert und übliche Zusatzstoffe wie Puffer-Substanzen, Detergentien, Aktivatoren oder Inhibitoren oder/und Stabilisatoren.

Mit Hilfe des Enzymimmunoassays sollen im allgemeinen Substanzen nachgewiesen werden, die in sehr geringer Konzentration vorliegen. Die Empfindlichkeit dieses Nachweissystems ist also von großer Bedeutung.

Bei Einsatz der neuen Substrate konnte in einem Enzymimmunoassay des Standes der Technik eine wesentliche Empfindlichkeitssteigerung erreicht werden. In diesem Assay wird die IgE-Konzentration als Maß für eine Allergie mit Hilfe der indirekten Antikörpertechnik bestimmt. Bei gleichen Inkubationszeiten für die verschiedenen Substrate konnte für Resorufin-$\beta$-Galactosid (Verb. S 6) eine etwa 4,3-fach höhere Extinktion und für 3-Hydroxy-2-nitropyridin-$\beta$-Galactosid eine etwa 1,7-fach höhere Extinktion als mit dem handelsüblichen o-Nitrophenyl-$\beta$-galactosid gemessen werden.

Bei gleicher Empfindlichkeit kann somit für das Resorufin-$\beta$-Galactosid die Inkubationszeit für die Enzymreaktion wesentlich verkürzt oder bei gleicher Inkubationszeit die Empfindlichkeit für den IgE-Nachweis erheblich gesteigert werden. Beide Schritte können von großer praktischer Bedeutung sein. Es hängt von der nachzuweisenden Substanz ab, ob eine größere Empfindlichkeit oder ein schnelleres Testergebnis wichtiger ist.

Tab. 7

| Bestimmung von IgE in derselben Probe mit einem Enzymimmunoassay des Standes der Technik bei Verwendung eines handelsüblichen und zweier neuer Substrate. | | |
|---|---|---|
| Substrat | Meßwellenlänge | Extinktionsänderung |
| Resorufin-$\beta$-galactosid | 576 nm | 0.761 |
| 3-Hydroxy-2-Nitropyridin-$\beta$-galactosid | 405 nm | 0.298 |
| o-Nitrophenylgalactosid | 405 nm | 0.176 |

Die folgenden Beispiele sollen die Erfindung erläutern.

## Beispiel 1

### 2H,7-0-($\alpha$-D-Glucopyranosyl)-4-methyl-8-nitrobenzpyran-2-on (Verbindung S 1)

17 g 1-$\beta$-Chlor-3,4,6-triacetylglucopyranosid wurden mit 15 g 2H,7-Hydroxy-4-methyl-8-nitrobenzpyran-2-on in Toluol während 20 h unter Rückfluß und Feuchtigkeitsausschluß erhitzt. Danach wurde das Lösungsmittel eingedampft, der Rückstand in $CH_2Cl_2$ aufgenommene, mit gesättigter $NaHCO_3$ Lösung gewaschen, getrocknet und wieder eingeengt. Der Syrup wurde in Methanol warm aufgenommen und auskristallisieren lassen. Ausbeute 22,5 g (75 % d. Th.)

Die Kristalle des Triacetylderivates wurden in 200 ml einer Mischung aus Methanol; Triäthylamin und Wasser im Verhältnis 50 : 5 : 5 suspendiert und gerührt, wobei sie sich nach etwa 40 min auflösten.
Nach 1-2 h schied sich das entacetylierte Glucosid aus, das über Nacht bei 0 °C stehen gelassen, abfiltriert und mit Methanol gewaschen wurde. Aus der Mutterlauge gewann man noch weitere Substanz.
Gesamtausbeute 11 g (65 % d. Th.); Schmelzpunkt: 130 °C.

| Elementaranalyse (Bruttoformel: $C_{16}H_{17}O_{10}N$; Molekulargewicht: 383,315): | | | | | | | | |
|---|---|---|---|---|---|---|---|---|
| berechnet<br>gefunden | C | 50,1<br>49,9 | H | 4,5<br>4,5 | H | 3,7<br>3,7 | O | 41,7<br>41,7 |

Beispiel 2

3-O-(α-D-Glucopyranosyl-)2-nitropyridin (Verbindung S2)

Die Verbindung S 2 wurde analog der Verbindung S 1 hergestellt mit der Ausnahme, daß 2H,7-Hydroxy-4-methyl-8-nitro-benz-pyran-2-on durch 10 g 3-Hydroxy-2-nitropyridin ersetzt wurde.
Schmelzpunkt: 178 ° C

| Elementaranalyse: | | | | | | | | |
|---|---|---|---|---|---|---|---|---|
| berechnet<br>gefunden | C | 43,7<br>43,7 | H | 4,7<br>4,8 | H | 9,4<br>9,1 | O | 42,2<br>42,5 |

Beispiel 3

3-O-(α-D-Galactopyranosyl)-2-nitropyridin(Verbindung S3)

Ein inniges Gemisch von 41 g (0,1 Mol) Acetobromgalactose und 28 g (0,2 Mol) 3-Hydroxy-2-nitropyridin wurde unter Feuchtigkeitsausschluß und gutem Rühren durch Erwärmen auf 70 ° C zusammengeschmolzen.
Nach Zugabe eines Gemischs von 13 g Hg $(CN)_2$ und 1 g $HgBr_2$ wurde unter Rühren (1 h) die Temperatur auf 100 ° gebracht (HCN Entwicklung). Nach Abkühlen wurden 50 ml Essigester und dann 200 ml $CCl_4$ zugegeben, mit 1 g Aktivkohle behandelt und dann durch 10 g Kieselgel durchfiltriert.
Das Filtrat wurde mit gesättigtem Bicarbonat und dann mit 1 n KBr-Lösung ausgeschüttelt, getrocknet und zur Trocknung verdampft. Der sirupöse Rückstand wurde in warmem Methanol gelöst und bei 0 ° kristallisieren gelassen.

Die Kristalle der Tetraacetylverbindung wurden in 200 ml Methanol/Triäthylamin/Wasser-Gemisch im Verhältnis 50/5/5 suspendiert und gerührt, wobei sie sich nach etwa 40 Minuten auflösten. Nach 1 - 2 Stunden schied sich das entacetylierte α-Galactosid aus. Es wurde über Nacht bei 0 ° C stehen gelassen, abfiltriert und mit Methanol gewaschen.
Schmelzpunkt: 220 ° C.

| Elementaranalyse (Bruttoformel): $C_{11}H_{14}O_8N_2$; Molekulargewicht: 302,243: | | | | | | | | |
|---|---|---|---|---|---|---|---|---|
| berechnet<br>gefunden | C | 43,7<br>43,8 | H | 4,7<br>4,8 | N | 9,2<br>9,2 | O | 42,3<br>42,8 |

Beispiel 4

2H,7-O-(β-D-Glucopyranosyl)-4-methyl-8-nitrobenzpyran-2-on (Verbindung S 4)

18 g 2H,7-Hydroxy-4-methyl-8-nitrobenzpyran-2-on + 16 g Acetobrom-glucose + 50 g wasserfreies $Na_2C_33$ wurden 20 h in 200 ml Aceton unter Rückfluß gekocht, abgefiltert, eingeengt, in Chloroform aufgenommen, mit 0,1 normaler NaOH ausgewaschen, getrocknet, wieder eingeengt und dann in Methanol aufgenommen, wobei das Tetraacetylderivat auskristallisierte.
Zur Entacetylierung rührte man das Tetraacetylderivat mit katalytischen Mengen Natriummethylat etwa 8 h in Methanol.
Schmelzpunkt: 210 ° C

| Elementaranalyse: | | | | | | | | |
|---|---|---|---|---|---|---|---|---|
| berechnet<br>gefunden | C | 50,1<br>50,4 | H | 4,5<br>4,6 | H | 3,7<br>3,7 | O | 41,7<br>41,2 |

Beispiel 5

3-O-(β-D-Glucopyranosyl)-2-nitropyridin (Verbindung S 5)

Die Verbindung S 5 wurde analog Verbindung S 4 hergestellt, wobei 2H,7-Hydroxy-4-methyl-8-nitrobenzpyran-2-on durch 16 g des Silbersalzes von 3-Hydroxy-2-nitropyridin ersetzt wurde.
Schmelzpunkt: 206 °C

| Elementaranalyse: | | | | |
|---|---|---|---|---|
| | C | H | H | O |
| berechnet:<br>gefunden: | 43,7<br>43,5 | 4,6<br>4,5 | 9,2<br>9,1 | 42,3<br>42,3 |

Beispiel 6

Resorufin-8-β-D-galactopyranosid (Verbindung S 6)

5 g Resazurin-Na wurden mit 8,2 g Acetobromgalactose während 5 Tagen in Acetonitril gerührt. Nach Abfiltrieren wurde das Lösungsmittel entfernt, der Rückstand in Essigester aufgenommen und mit gesättigter $NaHCO_3$-Lösung ausgeschüttelt. Nach dem Trocknen wurde auch der Essigester unter Vakuum entfernt und der Rückstand, bestehend aus Resazurin-β-galactosid-tetraacetat, aus Methanol auskristallisiert.
Resorufin-β-D-galactopyranosid wurde aus Resazurin-β-D-galactopyranosid-tetraacetat hergestellt durch Hydrieren in Methanol mit Hilfe von Palladium/Aktivkohle als Katalysator.

Als die Lösung vollständig farblos war, gab man 1 ml 0.1 n Natrium-Methylatlösung pro 100 ml zu, ließ 3 h rühren und filtrierte ab durch 10 g Kieselgel. Beim Einengen und Einblasen von Luft kristallisierte das Resorufingalactosid aus. Nach 24 h bei 0 °C saugte man die orangefarbenen Kristalle ab.
Schmelzpunkt: 222 °C

| Elementaranalyse: | | | | | | | | |
|---|---|---|---|---|---|---|---|---|
| berechnet<br>gefunden | C | 57,6<br>57,8 | H | 4,6<br>4,9 | N | 3,7<br>3,4 | O | 34,1<br>34,2 |

Beispiel 7

3-O-(β-D-Galactopyranosyl)-2-nitropyridin (Verbindung S7)

Die Verbindung S 7 wurde analog Verbindung S 4 hergestellt, wobei 2H,7-Hydroxy-4-methyl-8-nitrobenzpyran-2-on durch 16 g des Silbersalzes von 3-Hydroxy-2-nitropyridin und Acetobromglucose durch Acetobromgalactose ersetzt wurden.
Schmelzpunkt: 245 °C.

| Elementaranalyse: | | | | |
|---|---|---|---|---|
| | C | H | N | O |
| berechnet:<br>gefunden: | 43,7<br>43,7 | 4,7<br>4,6 | 9,4<br>9,3 | 42,2<br>42 1 |

Beispiel 8

2H,7-O-(2-Acetamido-2-deoxy-β-D-glucopyranosyl-)4-methyl-8-nitrobenzpyran-2-on (Verbindung S 8)

15 g β-chloro-N-acetylaminoglucosid wurden zusammen mit 6,63 g 2H,7-Hydroxy-4-methyl-8-nitrobenzpyran-2-on in 200 ml Aceton gelöst und nach Zugabe einer Lösung von 2 g NaOH in wenigen ml Wasser die Mischung einen Tag bei Raumtemperatur gerührt und weitere 5 Tage auf 0-5 ° C gehalten.
Das Aceton wurde im Vakuum entfernt, der Rückstand in $CHCl_3$ aufgenommen, mit gesättigter $NaHCO_3$-Lösung ausgeschüttelt; getrocknet und das $CHCl_3$ entfernt. Der Rückstand kristallisierte sofort; als er mit warmem Methanol behandelt wurde. Zur Entacetylierung benutzte man 1 ml 0,1 n Na-Methylatlösung in Methanol auf 100 ml Lösung.
Schmelzpunkt: 216-218 ° C

| Elementaranalyse: | | | | | | | | |
|---|---|---|---|---|---|---|---|---|
| berechnet<br>gefunden | C | 50,9<br>50,1 | H | 4,8<br>4,9 | N | 6,6<br>6,5 | O | 37,7<br>38,1 |

Beispiel 9

2H,7-O-(1-β-D-Glucuronopyranosyl)-4-methyl-8-nitrobenzpyran-2-on (Verbindung S 9)

In einem 1,5 l Rührkolben mit Intensivrührer wurden 15 g Verbindung S 4 in 300 ml Phosphat-Puffer pH 6,8 bei Raumtemperatur in Gegenwart von 2 g Platin-Mohr mit gasförmigem Sauerstoff während 72 h oxidiert. Am Ende wurde erwärmt, nach Abnutschen des Katalysators angesäuert, eingeengt und mit heißem Essigester extrahiert. Beim Stehen in Eis kristallisierte das gewünschte β-Glucuronid aus.

| Elementaranalyse: | | | | | | | | |
|---|---|---|---|---|---|---|---|---|
| berechnet<br>gefunden | C | 46,2<br>45,1 | H | 4,1<br>3,9 | N | 3,4<br>3,2 | O | 46,2<br>44,6 |

Beispiel 10

2H,7-O-(α-D-Maltotriosyl)-4-Methyl-8-nitrobenzpyran-2-on (Verbindung S 10)

Nach 2-3 Tagen Inkubation von 1 g der Verbindung S 1 und 5 g α-Cyclodextrin mit Cyclodextrin-glucosyl-transferase in Morpholin-ethansulfonsäure-Puffer bei pH 6,5 wurden die Transfer-Produkte an einer 150 x 5 cm Säule von Bio-Rad P 2 (400 mesh) bei 3-5 bar, 30 ° C und einer Fließgeschwindigkeit zwischen 120-250 ml/h chromatographiert. Elutionsmittel war entgastes Wasser.

Die verschiedenen Fraktionen wurden gesammelt und lyophilisiert.

| Elementaranalyse: | | | | | | | | |
|---|---|---|---|---|---|---|---|---|
| berechnet<br>gefunden | C | 45,5<br>45,3 | H | 5,5<br>5,3 | N | 1,8<br>1,9 | O | 47,3<br>44,5 |

Beispiel 11

2H,7-O-(α-D-Maltotetraosyl)-4-methyl-8-nitrobenzpyran-2-on (Verbindung S 11)

Verbindung S 11 wurde analog Verbindung S 10 hergestellt, wobei die nach der Verbindung S 10 von der Chromatographiesäule eluierte Fraktion gesammelt und lyophilisiert wurde.

HPLC-Analyse:

Auf einer Phenyl-Säule der Fa. Waters (Bondapak-Phenyl 10 µm, 3,9 x 300 mm) wurde mit einem Gemisch aus Wasser/Methanol im Verhältnis 70/30 und einer Fließrate von 1,12 ml/min eluiert. Die Verbindung S 11 wird unter diesen Bedingungen nach einer Elutionszeit von ca. 7,3 min eluiert.

Beispiel 12

3-0-(α-D-Maltotriosyl)-2-nitropyridin (Verbindung S 12)

Verbindung S 12 wurde analog Verbindung S 10 hergestellt, wobei an Stelle von Verbindung S 1 1 g der Verbindung S 2 eingesetzt wurde.

| Elementaranalyse: | | | | | | | | |
|---|---|---|---|---|---|---|---|---|
| berechnet<br>gefunden | C | 41,6<br>41,4 | H | 5,7<br>5,6 | O | 48,3<br>46,8 | N | 4,2<br>3,1 |

Beispiel 13

2H,7-O-(Phosphoryl)-4-Methyl-8-nitrobenzpyran-2-on (Verbindung S 13)

Alle Reagenzien müssen sorgfältig getrocknet sein. 12,5 g 2H,7-Hydroxy-4-methyl-8-Nitrobenzpyran-2-on wurden in 200 ml Pyridin gelöst. Die Lösung wurde tropfenweise unter Rühren zu einer Lösung von 8,5 g $POCl_3$ in 20 ml Pyridin gegeben. Hierbei wurde dafür gesorgt, daß die Temperatur 5 °C nicht übersteigt. Nach 30 Minuten wurde das Reaktionsprodukt durch Zufügen von Eiswasser (100 ml) zersetzt. Die Lösung wurde dann auf pH 7,5 gebracht, indem tropfenweise 10 N NaOH unter Rühren zugefügt wurde.
Pyridin und Wasser wurden unter reduziertem Druck bei möglichst tiefer Temperatur (50 °) abgedampft. Der Rückstand wurde mit 30 ml Aceton versetzt; verrieben und mit 125 ml warmem 80 Vol-%igem wässrigen Methanol extrahiert. Die Methanollösung wurde mit 300 ml Aceton versetzt. Man erhielt die Verbindung S 13 als Dinatriumsalz. Umkristallisieren aus 70 Vol-%igem wässrigem Äthylalkohol.

Beispiel 14

Resorufinphosphat (Verbindung S 14)

2,5 g (0,01 mol) Natrium-Resazurin in 50 ml trockenem Acetonitril wurden mit einer Lösung von 4,5 g Bis-2,2,2-trichlorethyl-phosphoryl-chlorid in 50 ml Acetonitril tropfenweise versetzt. 5 ml Pyridin wurden gegen Ende der Zugabe dazugegeben. Die Mischung wurde unter Eiskühlung 3 h und bei Zimmertemperatur weitere 15 Minuten gerührt. Die Mischung wurde im Vakuum eingeengt, der Rückstand in Chloroform aufgenommene mit $NaHCO_3$ gewaschen, getrocknet, eingeengt und in Ethanol kristallisiert. Resorufinphosphat erhielt man durch Reduktion des obigen Produkts mit Pyridin/Zinkstaub, wobei auch die Schutzgruppen entfernt wurden.

Beispiel 15

3H,7-Hydroxy-9-cyanxanthen-3-on(Cyanorufin; Verbindung C 1)

Zu einer eisgekühlten, gerührten Lösung von 9 g 3H,7-Hydroxy-9-hydroxyiminomethyl-xanthen-3-on und 11,5 ml wasserfreiem Pyridin in wasserfreiem Dioxan wurden 5,5 ml Trifluoressigsäureanhydrid

tropfenweise zugegeben in einer Geschwindigkeit, daß die Temperatur unter 5 °C bleibt. Nach der Zugabe wurde die Mischung auf Raumtemperatur gebracht und für weitere 6 h gerührt, auf 200 ml eisgekühlte 2N HCl gegeben, der ausgeschiedene Niederschlag abgenutscht, mit Wasser gewaschen und getrocknet. Umkristallisation aus Äthanol. Ausbeute 7,7 g (89,5 % d. Th.).
Der Schmelzpunkt des Cyanorufin liegt über 300 °C. Cyanorufin hat einen $pK_a$-Wert von 5,55, das Absorptionsmaximum liegt bei 578 nm, der molare Extinktionskoeffizient bei 578 nm und pH 7,1 ist 64.420 $1 \cdot mol^{-1} \cdot cm^{-1}$.

| Elementaranalyse (Bruttoformel $C_{14}H_7O_3N$, Molekulargewicht: 237,217: | | | | | | | | |
|---|---|---|---|---|---|---|---|---|
| berechnet<br>gefunden | C | 70,9<br>69,6 | H | 3,0<br>3,6 | O | 20,7<br>20,7 | N | 5,9<br>5,3 |

Die Vorteile dieses neuen Chromogens liegen in seinem niedrigen $pK_a$-Wert, in seinem sehr hohen Extinktionskoeffizienten und seiner Absorption im langwelligen Bereich.

Beispiel 16

Herstellung von N-Toluolsulfonyl-L-alanin (Tosylalanin)

0,01 Mol L-Alanin wurden in 20 ml 1-molarer Natronlauge gelöst, eine Lösung von 0,01 Mol p-Toluolsulfonsäurechlorid in 10 ml Diäthyläther hinzugefügt und die Mischung 4 h heftig gerührt. Die Ätherphase wurde abgetrennt, und die wässrige Phase mit konzentrierter Salzsäure auf pH 3 angesäuert. Das rohe N-Toluolsulfonyl-L-alanin begann zu kristallisieren (eventuell auf 4 °C abkühlen) und wurde nach dem Abfiltrieren aus 60 vol.-%igem, wässrigem Alkohol umkristallisiert. Fp. 130 - 131 °C.

Beispiel 17: Herstellung von Tosylalanylresorufin

In 5 ml Dimethylformamid (DMF) wurden 220 mg (1,1 mMol) Dicyclohexylcarbodiimid, 165 mg (1,1 mMol) 1-Hydroxybenzotriazol und 1 mMol Tosylalanin unter Rühren bei Raumtemperatur gelöst. Sodann wurden 210 mg (1 mMol) Resorufin (Fa. Aldrich, USA) hinzugegeben und weitere 2 h gerührt. Danach wurde die Mischung in 100 ml Eiswasser gegeben und mit 250 ml Essigsäureäthylester ausgeschüttelt. Nach Abtrennen wurde die Essigesterphase mit Natriumsulfat getrocknet und der Essigester im Vakuum abgezogen.
Das säulenchromatographisch gereinigte Produkt (Säule: Kieselgel; Laufmittel: $CHCl_3$ + $CH_3OH$ 9:1) hatte einen Schmelzpunkt von Fp = 165 °C und war dünnschichtchromatographisch rein.

Beispiel 18: Herstellung von Tos-gly-pro-arg-resazurin

1 mMol Tos-gly-pro-arg-OH wurden in 30 ml Tetrahydrofuran (trocken) suspendiert. 4 mMol Chlorameisensäureethylester wurden bei Raumtemperatur zugegeben, etwa 15 Minuten gerührt und auf - 10 °C abgekühlt. 3 mMol N-Methylmorpholin wurden zugegeben und 60 Minuten bei Raumtemperatur gerührt.
Dazu wurden 3 mMol Resazurin in 60 ml Tetrahydrofuran (trocken) getropft. Die Reaktion wurde nach 4 h beendet, abfiltriert und eingedampft. Das Rohprodukt wurde über eine Chromatographie-Säule gereinigt (stationäre Phase: Kieselgel; Laufmittel: $CHCl_3$ + Eisessig 19:1).
Ein Testpapier, das mit einer 0,1 %igen alkoholischen Lösung dieses Substrates getränkt und getrocknet wurde, reagiert beim Anfeuchten mit einer Blutplasma-Lösung unter sofortiger Blauviolettfärbung.

Beispiel 19

Bestimmung der alpha-Amylase-Aktivität mit der Verbindung S 10

In 100 ml $^{m}/_{15}$ Phosphat-Puffer, pH 6,0, werden 585 mg NaCl und 200 mg Verbindung S 10 gelöst.
In 1 ml dieser Lösung wurden 20 µl Harn pipettiert und gut vermischt. In einem Photometer mit angeschlossenem Schreiber wurde bei 365 nm die Extinktionsänderung kontinuierlich aufgezeichnet. Anhand der Schreiberaufzeichnung wird die Extinktionsänderung pro Minute (deltaE/min) berechnet und die Enzymaktivität (U/l) mit folgender Formel errechnet:

$$U/l = \frac{deltaE/min \times 1{,}02 \times 1000}{0{,}02 \times 16{,}1}$$

$$= deltaE/min \times 3168$$

1,02 = Testvolumen inclusive Probe in ml
0,02 = Probevolumen in ml
1000 = Umrechnungsfaktor von ml in l
16,1 = Extinktionskoeffizient für 2H,7-Hydroxy-4-methyl-8-nitrobenzpyran-2-on bei pH 6,0 und 365 nm in $cm^2/\mu mol$
Andere Hydrolasen können mit einem der erfindungsgemäßen und für das Enzym spezifischen Substrate bei angepaßten Reaktionsbedingungen analog bestimmt werden.

Beispiel 20

Bestimmung der Leukozyten-Esterase mit Tosyl-alanyl-resorufin

Das Indikatorpapier Nr. 218 von Macherey-Nagel, Düren, Bundesrepublik Deutschlands wurde nacheinander mit folgenden Lösungen getränkt und getrocknet:
a) wäßriger Borsäurepuffer 0,25 mol/l, pH 8
b) Tosyl-alanyl-resorufin 0,25 g in 1 l Essigsäureethylester gelöst.
Als Trocknungstemperatur für die Lösung a) wurde etwa 80 °C und für b) etwa 120 °C gewählt.
Als Proben wurden Suspensionen mit verschiedenen Leukozyten-Konzentrationen eingesetzt, in die die Teststreifen kurz eingetaucht wurden. Nach etwa 1 Minute entwickelte sich in Abhängigkeit von der Leukozyten-Konzentration eine mehr oder weniger intensive rot-violette Farbe. Die Nachweisempfindlichkeit dieses Testsystems liegt bei 1000 Leukozyten pro Mikroliter.

Durch Einsatz von N-Tosyl-aminobutyryl-oder Boc-Leu-Resorufin in Tränklösung b) wurde ein Testpapier erhaltene, dessen Nachweisempfindlichkeit bei 1000 bzw. 2000 Leukozyten pro Mikroliter liegt.

Beispiel 21

Herstellung von 2-H-7-0($\beta$-Galactopyranosyl-)4-methyl-8-nitrobenzpyran-2-on, Verbindung S 15

Die Verbindung S 15 wurde analog der Verbindung S 4 hergestellt, nur daß an Stelle der Acetobromglucose 16 g Acetobromgalactose eingesetzt wurden.
Schmelzpunkt: 188 - 192 °C

| Elementaranalyse: | | | | |
|---|---|---|---|---|
| | C | H | N | O |
| berechnet: | 50,1 | 4,5 | 3,7 | 41,7 |
| gefunden: | 50,2 | 4,6 | 3,7 | 41,3 |

**Patentansprüche**

1. Verbindung der allgemeinen Formel A-O-B, worin A den Rest des Ions $A\text{-}O^-$ einer heterocyclischen Verbindung A-OH mit saurem $pK_a$-Wert darstellt, dessen Extinktion nach Hydrolyse von A-O-B photometrisch gemessen wird, während B der Rest einer Verbindung B-OH ist, der die Verbindung für die Reaktion mit einem bestimmten Enzym spezifisch macht, und wobei A-OH

a. eine Verbindung der allgemeinen Formel I ist

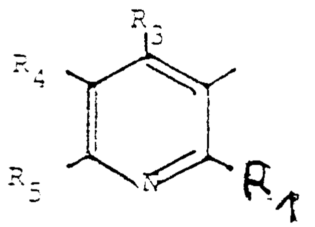

I

worin $R_1$ OH, $NO_2$ oder Halogen und
$R_3$, $R_4$ und $R_5$ H oder $NO_2$ sein können, wobei höchstens zwei $NO_2$ vorhanden sind,
oder
b. eine Verbindung der allgemeinen Formel IV ist

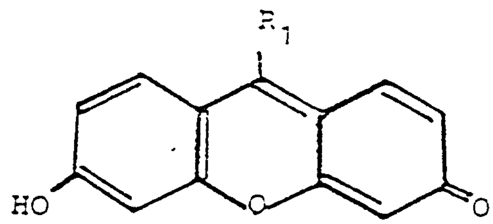

IV

worin $R_1$ Aryl, vorzugsweise Phenyl, CN oder Alkyl mit 1-12, vorzugsweise 1-3, Kohlenstoffatomen sein können, und wobei B-OH ein Zucker oder Zuckerderivat, insbesondere Glucose, ein Maltodextrin, Galactose, Glucuronsäure, N-Acetylglucosamin oder Fucose, eine Aminosäure oder ein Oligopeptid, die eine Schutzgruppe, besonders die Toluolsulfonyl- oder Carbobenzyloxy (CBZ)-Gruppe tragen können, insbesondere N-Tosylalanin oder CBZ-Glycin, oder eine anorganische Säure, insbesondere Phosphorsäure ist, wobei Schwefelsäure ausgenommen ist.

**2.** Verfahren zur Herstellung einer Verbindung der Formel A-O-B nach Anspruch 1, dadurch gekennzeichnet, daß man eine Verbindung der Formel A-OH mit einem reaktionsfähigen Derivat einer Verbindung der Formel B-OH reagieren läßt.

**3.** Verwendung einer Verbindung der Formel A-O-B nach Anspruch 1 als Substrat zum Nachweis und zur Bestimmung einer Hydrolase.

**4.** Diagnostisches Mittel zum Nachweis esterolytischer Enzyme, insbesondere zum Nachweis der in Leukozyten vorhandenen Esterasen bestehend aus einem saugfähigen Träger, einer Filmschicht, einer losen oder verpreßten Pulvermischung, einem Lyophilisat oder einer Lösung, enthaltend eine Verbindung der Formel A-O-B nach Anspruch 1, in der AOH ein Farbstoff der allgemeinen Formel IV und BOH eine Aminosäure oder ein Oligopeptid, gegebenenfalls mit einer Schutzgruppe versehen ist, und übliche Zusatzstoffe wie Puffer-Substanzen, Detergentien, Aktivatoren oder Inhibitoren oder/und Stabilisatoren.

## Claims

**1.** A compound corresponding to the general formula A-O-B, wherein A is a moiety of an anion $A\text{-}O^-$ originating from the heterocyclic compound A-OH having an acidic $pK_a$ and the extinction of which is measured photometrically after hydrolysis of A-O-B, and wherein B is a moiety of a compound B-OH rendering said compound capable for reacting with a certain enzyme, said A-OH being

a. a compound corresponding to the general formula I

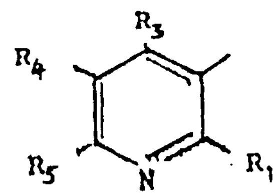

I

wherein $R_1$ may be OH, $NO_2$ or Halo and
$R_3$, $R_4$ and $R_5$ may be H, $NO_2$, and not more than two $NO_2$ are present,
or
b. a compound corresponding to the general formula IV

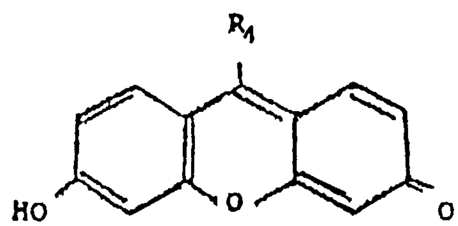

IV

wherein $R_1$ can be aryl, preferably phenyl, CN or alkyl having 1-12 carbon atoms, preferably 1-3 carbon atoms, and
said B-OH being a sugar or a sugar derivative, especially glucose, maltodextrins, galactose, glucuronic acid, N-acetylglucosamine or fucose, an amino acid or an oligopeptide which may be capped especially using a toluenesulfone group or a benzeneoxycarbonyl (BOC) group, preferably N-tosylamine or BOC-glycine, or an inorganic acid, especially phosphoric acid, but not being sulfuric acid.

**2.** A method for preparation of a compound of the formula A-O-B according to claim 1 characterized in that a compound of the formula A-OH is reacted with a reactive derivative of a compound corresponding to the formula B-OH.

**3.** The use of a compound corresponding to the formula A-O-B according to claim 1 as substrate for detection and determination of a hydrolase.

**4.** A diagnostic agent for detection of esterolytic enzymes, especially esterases present in leucocytes, consisting of an absorptive carrier, a film layer, a compacted or uncompacted mixture of powders, a lyophilisate or a solution containing a compound corresponding to the formula A-O-B according to claim 1, wherein AOH is a colorant of the general formula IV and BOH is an amino acid or an oligopeptide optionally capped, and usual additives such as buffering substances, detergents, activating or inhibiting substances or/and stabilizers.

**Revendications**

**1.** Composé de formule générale A-O-B, dans laquelle A représente le résidu de l'ion A-$O^-$ d'un composé hétérocyclique A-OH avec une valeur $pK_a$ acide dont l'extinction est mesurée photométriquement après l'hydrolyse de A-O-B, tandis que B est le résidu d'un composé B-OH qui rend le composé spécifique pour la réaction avec une enzyme déterminée, A-OH étant

a. un composé de formule générale I

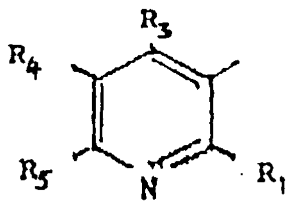

I

dans laquelle $R_1$ peut être OH, $NO_2$ ou un halogène et
$R_3$, $R_4$ et $R_5$ peuvent être H ou $NO_2$, deux $NO_2$ au plus étant présents
ou
b. un composé de formule générale IV

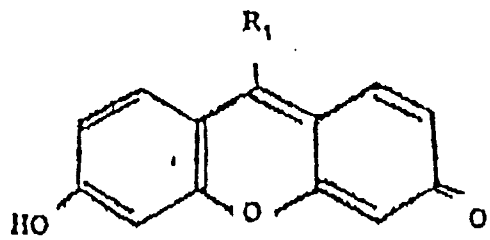

IV

dans laquelle $R_1$ peut être un radical aryle, de préférence un radical phényle, CN ou alkyle avec 1 à 12, de préférence 1 à 3 atomes de carbone, B-OH étant un sucre ou un dérivé du sucre, en particulier le glucose, une maltodextrine, le galactose, l'acide glucoronique, la N-acétylglucosamine ou le fucose, un acide aminé ou un oligopeptide qui peuvent porter un radical de protection, en particulier le radical toluolsulfonyle ou carbobenzyloxy (CBZ), en particulier la N-tosylalanine ou la CBZ-glycine ou un acide inorganique, en particulier l'acide phosphorique, à l'exception de l'acide sulfurique.

**2.** Procédé de préparation d'un composé de formule A-O-B selon la revendication 1, caractérisé en ce qu'un composé de formule A-OH est amené à réagir avec un dérivé réactif d'un composé de formule B-OH.

**3.** Mise en oeuvre d'un composé de formule A-O-B selon la revendication 1 comme substrat en vue de l'identification et de la détermination d'une hydrolase.

**4.** Moyen diagnostique en vue de l'identification d'enzymes estérolytiques, en particulier de l'identification d'estérases présentes dans des leucocytes, composé d'un support absorbant, d'une couche en pellicule, d'un mélange de poudre libre ou compacte, d'un produit lyophilisé ou d'une solution contenant un composé de formule A-O-B selon la revendication 1, dans lequel AOH est un colorant de formule générale IV et BOH est un acide aminé ou un oligopeptide, portant éventuellement un groupe de protection et des additifs habituels, tels que des substances tampons, des détergents, des activateurs ou inhibiteurs et/ou stabilisateurs.